# EUROPÄISCHE PATENTSCHRIFT

(11) **EP 2 777 708 B1**
(45) Veröffentlichungstag und Bekanntmachung des Hinweises auf die Patenterteilung: **02.11.2016**
(21) Anmeldenummer: 14159181.8
(22) Anmeldetag: 12.03.2014
(51) Int. Cl.: A61K 33/14, A61K 9/00, A61K 33/40, A61P 1/02

(54) **Pharmazeutisches Mittel zur Behandlung von Aphthen**
Pharmaceutical composition for the treatment of mouth ulcers
Produit pharmaceutique destiné au traitement des aphtes

(30) Priorität: 12.03.2013 CH 5842013
(43) Veröffentlichungstag der Anmeldung: 17.09.2014
(73) Patentinhaber: Bonyf AG, 9490 Vaduz (LI)
(72) Erfinder: Bogaert, Jean-Pierre, Monaco (MC)
(74) Vertreter: Riederer Hasler & Partner Patentanwälte AG

(56) Entgegenhaltungen:
- WO-A1-99/34773
- WO-A2-2004/000372
- US-A- 4 758 630
- US-A- 5 561 177
- US-A1- 2012 148 506
- US-A1- 2012 296 006
- US-A1- 2013 197 124

## Beschreibung

### Technisches Gebiet der Erfindung

Die vorliegende Erfindung betrifft ein pharmazeutisches Mittel zur Behandlung von Aphthen.

### Stand der Technik

Eine Aphthe ist gemäss dem online-Lexikon Wikipedia eine schmerzhafte, von einem entzündlichen Randsaum umgebene Schädigung der Schleimhaut des Zahnfleischs, der Mundhöhle, der Tonsillen oder der Zunge. Es handelt sich dabei um ein Ulcus mit weißlichem Fibrinbelag.

Zur symptomatischen Behandlung von Aphthen stehen Sprays, Gurgellösungen und Gele bzw. Salben (Haftsalben für die Mundschleimhaut) für die Therapie zur Verfügung. Eine prophylaktische Behandlung von Aphthen ist nicht möglich, weil die Ursachen für die Entstehung von Aphthen im Gaumen nicht bekannt sind.

Aus der Offenbarung WO 2004/000372 ist eine Zusammensetzung bekannt, welche eine salzförmige Chlorid- oder Bromidverbindung, mindestens ein Oxidationsmittel mit einem Oxidationspotential, welches in Lösung höher als das Oxidationspotential von Cl¹⁻/Cl⁰ resp. Br¹⁻/Br⁰ ist, und im Überschuss mindestens eine Säure zur Erzeugung eines pH-Wertes < 6 bei vollständig aufgelöster Zusammensetzung enthält. Diese Zusammensetzung dient vorwiegend zur Desinfektion und Reinigung von dentalen Gegenständen, wie Zahnbürsten und künstlichen Gebissen, und Rasiergeräten sowie Körperteilen, wie Finger- und Zehennägel, kann jedoch auch als topisch wirkendes Mittel im Gaumen und Rachenbereich verwendet werden. Diese Zusammensetzung wird insbesondere in Form von selbstauflösenden Tabletten oder Granulaten eingesetzt.

US Patentanmeldung Nr. US 2012/0148506 offenbart eine antiseptische, antimikrobielle Zusammensetzung bestehend aus einem Gemisch aus Wasserstoffperoxid, Eugenol, natürlichem Kampfer, Zinksulfat, Natriumfluorid, Xylit, Cetylpyridiniumchlorid und Hilfsstoffen, welche frei von Phenol- oder Chlorophenol-Verbindungen ist, zur oralen Anwendung und zur Behandlung von Munderkrankungen bakterieller, mykotischer oder viraler Ätiologie. Diese Zusammensetzung wird z.B. in Form einer Mundspülung, eines Gel oder einer Zahnpaste angewendet. Unter anderem wird die Behandlung von und Vorbeugung gegen orale Aphthen vorgeschlagen.

WO 99/34773 offenbart eine Zusammensetzungen, umfassend ein Reaktionsgemisch bestehend aus erstens einer Peroxiverbindung oder pharmazeutisch annehmbarer Salze oder Ester davon, zweitens einer Ionenquelle, ausgewählt aus der Gruppe bestehend aus Chlorid-Ion, Bronmid-Ion, Iodid-Ion, Thiocyanat-Ion und Mischungen davon und drittens einen topischen Träger, wie z.B. Gel, Mundspülung, Zahnpaste, Zahnpulver, Mundspray, Kaugummi, Pastillen, Sachets, und Bleich-Kits usw. für die Behandlung topisch behandelbarer mikrobieller Infektionen, insbesondere von Erkrankungen der Mundhöhle, wie Plaque, Gingivitis und Parodontitis, Mundgeruch, und um die Zähne aufzuhellen. Im Weiteren wird die Behandlung von oralen Aphthen vorgeschlagen.

Aus der Offenbarung EP 1 886 664 A1 ist eine Zusammensetzung umfassend eine wässrige N-Chloramine-Lösung und ein anionisches oberflächenaktives Mittel bekannt, welche als Zahnpaste, Mundspülung, therapeutisches Gel, Konservierungslösung oder Reinigungscreme formuliert ist.

### Aufgabe der Erfindung

Es ist Aufgabe der vorliegenden Erfindung, ein verbessertes Mittel zur Behandlung von Aphthen zur Verfügung zu stellen. Insbesondere soll eine einfache, lokalisierte und nachhaltige Behandlung ermöglicht werden.

### Beschreibung der Erfindung

Gegenstand der vorliegenden Erfindung ist ein Mittel gemäss Oberbegriff von Anspruch 1, welches dadurch gekennzeichnet ist, dass die pharmazeutische Zusammensetzung wenigstens eine organische oder anorganische Chlorverbindung und wenigstens ein Oxidationsmittel, welches ausgewählt ist aus der Gruppe der Persulfatverbindungen, enthält. Diese Zusammensetzung hat den Vorteil, dass nach der Applikation des Mittels auf einer entzündeten Stelle der Mundschleimhaut sich durch den Kontakt mit Speichel (Wasser) Chloroxid oder Hypochlorit bildet, welches für die heilende Wirkung des Mittels verantwortlich zu sein scheint. Die Haftzusammensetzung bildet dabei eine Barriereschicht, in welcher gleichzeitig die pharmazeutisch aktiven Stoffe enthalten sind. Diese entfalten ihre Wirkung jedoch erst, wenn Feuchtigkeit zu diesen gelangt.

Im Rahmen der vorliegenden Beschreibung sind mit dem Begriff "Zusammensetzung" jeweils die Komponenten der pharmazeutisch wirksamen Verbindungen gemeint. Unter dem Begriff "Haftzusammensetzung" sind hingegen jene Komponenten gemeint, welche als Träger (carrier) und Applikationsmedium für die pharmazeutische Zusammensetzung dienen. Die Haftzusammensetzung hat aber auch den Zweck, eine Barriereschicht über der Aphthe zu bilden und die Wirkstoffe erst allmählich freizusetzen. Eine lokalisierte Auftragung und Haftung der Haftzusammensetzung ermöglicht dabei einen lokalen und somit zielgenauen Einsatz der Wirkstoffe.

Das Chloratom kann in der Ausgangsverbindung entweder in Form eines Alkali- oder Erdalkalimetallsalzes oder einer anderen, in wässeriger Lösung Chloridionen freisetzenden Verbindung vorliegen, oder an einen organischen Rest gebunden sein. Organische Moleküle, welche Chloridionen freisetzen können, sind beispielsweise Chloramin B (N-Chlorbenzolsulfonamido-Natrium), Chloramin T (p-Toluolsulfonchloramin-Natrium), Dichloramin T (p-Toluolsulfondichloramid), Halazon (p- Dichlorsulfamylbenzoesäure), Dichlorcyanursäure, Trichlorcyanursäure, TCM (Trichlormelamin),1,3-Dichlor-5,5-dimethylhydantoin, Dichlorglycoluril, Succinchlorimid oder Chloroazodin (N,N'-dichlorodiazodicarbonamidin). Auch kann eine Mischung der vorgenannten chlorhaltigen Stoffe eingesetzt sein.

Das erfindungsgemässe Mittel zeichnet sich vorzugsweises dadurch aus, dass keine Fluoridsalze, vorzugsweise insgesamt keine Fluoridverbindungen, enthalten sind.

Vorteilhaft ist ein Oxidationsmittel eingesetzt, welches in wässeriger Lösung von der Chlorverbindung Chlor oder Chloroxid abzuspalten vermag. Geeignete Oxidationsmittel sind beispielsweise Persulfatverbindungen, insbesondere Hydrogenperoxosulfat-Verbindungen. In Verbindung mit wenigstens einem geeigneten Oxidationsmittel kann durch Reaktion mit gelösten Chloridionen (Cl⁻) Chlor (Cl₂) oder Dichloroxid (Cl₂O) gebildet werden. Der Vorteil einer derartigen Zusammensetzung ist, dass das Chlor nach und nach freigesetzt wird. Vorteilhaft ist das Oxidationspotential des Oxidationsmittels in Lösung mindestens beim bevorzugten pH-Bereich < 6, resp. < 5 höher als das Oxidationspotential von Cl⁻/Cl^{o}, damit die erwünschte Reaktion eintreten kann.

Obwohl grundsätzlich unterschiedliche Oxidationsmittel eingesetzt werden können, ist in der Zusammensetzung als Oxidationsmittel vorteilhaft eine Persulfatverbindung, insbesondere eine Hydrogenperoxosulfatverbindung enthalten. Persulfatverbindungen umfassend Peraxomonosulfatverbindungen (d.h. Verbindungen beinhaltend das Anion [SO₅]²⁻ ) und Peroxodisulfatverbindungen (d.h. Verbindungen beinhaltend das Anion [S₂O₈]²⁻). Beispiele dieser Verbindungen sind Peroxomonosulfate wie Na₂SO₅ oder KHSO₅ und Peroxodisulfate wie Na₂S₂O₈. Besonders bevorzugt als Oxidationsmittel sind die Hydrogenperoxosulfat-verbindungen. Ein vorteilhaftes Mittel erhält man mit Kaliumhydrogenmonopersulfat (KHSO₅) als Oxidationsmittel und einer salzförmigen Chloridverbindung, vorzugsweise aus der Gruppe der Alkalisalze, z.B. Kochsalz (NaCl). Dieses hat sich als besonders wirksam erwiesen.

Zweckmässigerweise enthält das Mittel noch eine Carbonsäure, vorzugsweise eine Mono- oder Dicarbonsäure wie Zitronensäure. Diese organischen Carbonsäuren sorgen für einen sauren pH-Wert, welcher die Wirksamkeit des Mittels noch verbessern kann.

Eine vorteilhafte Zusammensetzung, welche kostengünstig herstellbar ist, enthält Kaliumhydrogenmonopersulfat (KHSO₅), Kochsalz (NaCl) und eine Säure, vorzugsweise in Form einer Monocarbonsäure oder einer Dicarbonsäure wie Oxalsäure, Weinsäure, Bernsteinsäure oder Zitronensäure. Es ist vorzugsweise eine solche Menge Säure in der Zusammensetzung enthalten, dass nach dem Auflösen der Zusammensetzung im wässerigen Milieu (5 g Zusammensetzung in 50 ml Wasser) ein pH-Wert < 5, vorzugsweise < 4.5 erreicht ist. Zitronensäure ist physiologisch völlig unbedenklich, wird in der Natur rasch abgebaut und daher bevorzugt in der Zusammensetzung eingesetzt.

Die Zusammensetzung kann zusätzlich keimtötende Verbindungen, wie z.B. Kaliummonopersulfat, Kalium-Caroate, Natriumperoxycarbonat, Natriumbenzoat, Subtilisin, Kaliumbenzoat, Chlorhexidin, eine Kombination von Chlorhexidin und Thymol, Cetylpyridiniumchlorid, Halogen abspaltende Verbindungen wie PVP-Jod und Cyanursäurechlorid, und/oder Formaldehyd abspaltende Verbindungen wie Paraformaldehyd und/oder Methylolverbindungen etc. enthalten. Vorteilhaft sind zudem schmerzstillende Wirkstoffe wie beispielsweise Lidocain, Polidocanol oder Benzydamin eingesetzt.

Zweckmässigerweise enthält die Zusammensetzung ein Bindemittel, und optional Aroma-, Farb- und Hilfsstoffe wie Stoffe zur Wasserenthärtung, Füllstoffe und dergleichen. Als Bindemittel kann beispielsweise modifizierte Maisstärke, mikrokristalline Cellulose, Sorbitol, hydrierte Soyatriglyceride, Polyethylenglykol wie PEG 180, PEG 150, PEG 75, Polyvinylpyrrolidone, ein Copolymer von Ethylenoxid und Propylenoxid, Polyvinylpyrolidon oder ein Copolymer von Polyvinylpyrolidon Vinylacetat, etc. eingesetzt werden. Der gewichtsmässige Anteil des Bindemittels beträgt zweckmässigerweise maximal ungefähr 30% und liegt vorzugsweise zwischen 5 und 25% des Gesamtgewichts der pharmazeutischen Zusammensetzung.

Die Haftzusammensetzung enthält vorzugsweise wenigstens ein wasserlösliches Polymer, vorzugsweise ausgewählt aus der Gruppe der Cellulosederivate, wenigstens ein Alkylvinylether/Maleinsäureanhydrid-Copolymer, und optional raffinierte Paraffine (Vaselin), Fette und/oder Öle. Die Haftzusammensetzung kann weiter optional zinkhaltige Substanzen, Siliziumdioxid und Polyethylenglykol enthalten.

Vorteilhaft ist die pharmazeutische Zusammensetzung in Form eines pulverförmigen Feststoffgemisches der Haftzusammensetzung beigemischt. Diese Form der Beimengung kann einen retardierenden Effekt auf die Wirkstoffentfaltung haben, indem z.B. erst durch den allmählichen Kontakt mit dem Speichel die Auflösung der pulverförmigen pharmazeutischen Zusammensetzung erfolgt.

In der Zusammensetzung kann optional ein Tensid enthalten sein. Grundsätzlich können unterschiedliche, dem Fachmann bekannte Tenside eingesetzt werden. Wenn Carbonsäuren eingesetzt sind, so sollen die Tenside bei einem pH-Wert kleiner 7 stabil sein. Unter "stabil" soll in diesem Zusammenhang verstanden werden, dass max. 10% des eingesetzten Tensids in Lösung bei Raumtemperatur innerhalb von 30 Minuten zerfallen. Vorzugsweise werden als Tenside Fettalkohol-Polyglykoläther, Alkylbenzolsulfonate, Alkylsulfonate eingesetzt. Besonders bewährt haben sich anionische Tenside, vorzugsweise aus der Gruppe der Alkylethersulfate, wie Fettalkoholethersulfat-Alkalisalze, z.B. Natrium-n-alkyl-C₁₂₋₁₄-diglykoläthersulfat oder Natrium-n-alkyl-C₁₂₋₁₄-diglykoläthersulfat. Weiter eignen sich beispielsweise Na-Laurylsulfat, Na-Laurylsulfoacetat, Trinatriumphosphat.

### Beispiel:

**Eine beispielhafte pharmazeutische Zusammensetzung enthält folgende Komponenten:**

| **Stoff** | **in Gew.-proz. (%)** | **bevorzugte Bereiche in Gew.-proz. (%)** |
|---|---|---|
| Kaliumhydrogen-Monopersulfat | 5 | 1 bis 10 |
| Laurylsulfat | 15 | 2 bis 20 |
| Natrium-bicarbonat | 20 | 10 bis 40 |
| Zitronensäure | 30 | 15 bis 50 |
| Bindemittel | 20 | 5 bis 30 |
| Natriumchlorid | 10 | 4 bis 20 |

Zur Herstellung des pharmazeutischen Mittels wird die pharmazeutische Zusammensetzung in einer Haftcrème oder einem Haftgel dispergiert und fein verteilt, bis eine homogene Mischung erreicht ist. Dabei werden die Haftzusammensetzung und die pharmazeutische Zusammensetzung in einem gewichtsmässigen Verhältnis zwischen 99 :1 und 80 : 20, vorzugsweise 98 : 2 und 88 : 12 und besonders bevorzugt im Verhältnis 96 : 4 und 92 : 8 miteinander gemischt.

Haftzusammensetzungen, welche als Trägermaterial für die pharmazeutische wirksame Zusammensetzung dienen sind in den folgenden Patenten und Patentanmeldungen erwähnt: US Patentanmeldung Nr.13/574,230, US Patentanmeldung Nr. 13/574,224, US 4,758,630 und US 5,561,177.

Zur Anwendung durch den Benutzer/Patienten wird ein Pfropfen des viskosen Mittels auf die Aphthe aufgetragen und darüber verteilt, damit auch die Randzone der Aphthe gut abgedeckt ist. Das Mittel bildet auf der feuchten Mundschleimhaut eine Barriere- oder Schutzschicht. Gleichzeitig entfalten die in der Schutzschicht vorhandenen pharmazeutisch aktiven Ingredienzen ihre Wirkung. Durch die von Mundschleimhaut stammenden Feuchte und den im Mund vorhandenen Speichel werden die im Mittel vorhandenen pulverförmigen Komponenten gelöst, sodass diese miteinander reagieren können. Weil diese Komponenten nur langsam in Lösung gehen, kann eine gute Langzeitwirkung erzielt werden.

Ein Mittel zur Behandlung von Aphthen umfasst eine fliessfähige, pastöse Haftzusammensetzung, welche geeignet ist, nach Applikation auf der Mundschleimhaut zu haften, und eine pharmazeutische Wirkzusammensetzung mit einer organischen oder anorganischen Chlorverbindung und wenigstens einem Oxidationsmittel. Wenn die organische oder anorganische Chlorverbindung und das Oxidationsmittel in Lösung gehen, bildet sich Chlor oder Chloroxid, welches sich überraschenderweise besonders wirksam bei der Behandlung von Aphthen herausgestellt hat. Vorteilhaft enthält die Zusammensetzung noch eine organische Carbonsäure, um einen pH-Wert < 6 einzustellen.

## Patentansprüche

1. Mittel zur Behandlung von Aphthen, umfassend
- eine fliessfähige, pastöse Haftzusammensetzung, welche geeignet ist, nach Applikation an der Mundschleimhaut zu haften, und
- einer in der Haftzusammensetzung enthaltenen pharmazeutischen Zusammensetzung zur Behandlung der Entzündung
**dadurch gekennzeichnet,**
**dass** die pharmazeutische Zusammensetzung
- wenigstens eine organische oder anorganische Chlorverbindung und
- wenigstens ein Oxidationsmittel enthält, welches ausgewählt ist aus der Gruppe der Persulfatverbindungen.

2. Mittel zur Verwendung gemäss Anspruch 1, **dadurch gekennzeichnet, dass** eine Carbonsäure, vorzugsweise eine Mono- oder Dicarbonsäure wie Zitronensäure, enthalten ist.

3. Mittel zur Verwendung gemässAnspruch 2, **dadurch gekennzeichnet, dass** die Säure in einer solchen Menge in der Zusammensetzung enthalten ist, dass die in einer bestimmten Menge einer wässerigen Lösung aufgelöste Zusammensetzung
- einen pH-Wert < 6, vorzugsweise < 5, und ganz besonders bevorzugt < 4.5 erzeugt.

4. Mittel zur Verwendung gemässeinem der Ansprüche 1 bis 3, **dadurch gekennzeichnet, dass** als Oxidationsmittel eine Hydrogenperoxosulfatverbindung enthalten ist.

5. Mittel zur Verwendung gemässeinem der Ansprüche 1 bis 4, **dadurch gekennzeichnet, dass** die Chlorverbindung in Form eines Alkali- oder Erdalkalimetallsalzes vorliegt.

6. Mittel zur Verwendung gemäss einem der Ansprüche 1 bis 5, **dadurch gekennzeichnet, dass** die Chlorverbindung Chloramin B (N-Chlorbenzolsulfonamido-Natrium), Chloramin T (p-Toluolsulfonchloramin-Natrium), Dichloramin T (p-Toluolsulfondichloramid), Halazon (p-Dichlorsulfamylbenzoesäure), Dichlorcyanursäure, Trichlorcyanursäure, TCM (Trichlormelamin), 1,3-Dichlor-5,5-dimethylhydantoin, Dichlorglycoluril, Succinchlorimid oder Chloroazodin (N,N'-dichlorodiazodicarbonamidin) oder eine Mischung dieser Stoffe ist.

7. Mittel zur Verwendung gemäss einem der Ansprüche 1 bis 6, **dadurch gekennzeichnet, dass** die Zusammensetzung wenigstens Kaliumhydrogenmonopersulfat (KHSO₅) als Oxidationsmittel und, eine salzförmige Chloridverbindung, vorzugsweise aus der Gruppe der Alkalisalze, z.B. Kochsalz (NaCl), als Chlorquelle enthält.

8. Mittel zur Verwendung gemäss einem der Ansprüche 1 bis 7, **dadurch gekennzeichnet, dass** das Oxidationspotential des Oxidationsmittels in wässeriger Lösung mindestens im beanspruchten pH-Bereich höher als das Oxidationspotential von Cl¹⁻/Cl^{o} ist.

9. Mittel zur Verwendung gemäss einem der Ansprüche 1 bis 8, **dadurch gekennzeichnet, dass** die pharmazeutische Zusammensetzung in Form eines pulverförmigen Feststoffgemisches der Haftzusammensetzung beigemischt ist.

10. Mittel zur Verwendung gemäss einem der Ansprüche 1 bis 9, **dadurch gekennzeichnet, dass** die Zusammensetzung Mittel zur Lösungsbeschleunigung (Brausesalze), beispielsweise eine Carbonat- oder Bicarbonat enthaltende Verbindung wie z.B. Natriumcarbonat oder Natriumbicarbonat, und eine mindestens stöchiometrische Menge Säure enthält.

11. Mittel zur Verwendung gemäss einem der Ansprüche 1 bis 10, **dadurch gekennzeichnet, dass** das Mittel ein Bindemittel, und optional Aroma-, Farb- und Hilfsstoffe wie Stoffe zur Wasserenthärtung und Füllstoffe, enthält, und als Haftsalbe oder Gel vorliegt.

12. Mittel zur Verwendung gemäss einem der Ansprüche 1 bis 11, **dadurch gekennzeichnet, dass** die Haftzusammensetzung
wenigstens ein wasserlösliches Polymer, vorzugsweise ausgewählt aus der Gruppe der Cellulosederivate,
wenigstens ein Alkylvinylether/Maleinsäureanhydrid-Copolymer enthält.

13. Mittel zur Verwendung gemäss Anspruch 12, **dadurch gekennzeichnet, dass** die Haftzusammensetzung Paraffine, Fette und/oder Öle enthält.

14. Mittel zur Verwendung gemäss einem der Ansprüche 1 bis 13, **dadurch gekennzeichnet, dass** keine Fluoridsalze, vorzugsweise keine Fluoridverbindungen, enthalten sind.

## Claims

1. Means for the treatment of aphtae comprising:
- a flauvable pasty adhesive composition that is able to adhere to the mucous membrane of the oral cavity after application and
- a pharmaceutical composition contained in the adhesive composition for the treatment of the inflammation,
**characterized in that** the pharmaceutical composition contains
- at least one organic or inorganic chloric compound and
- at least one oxidant that is selected from the group of the persulfate compounds.

2. Means for the treatment according to claim 1, **characterized in that** it contains a carboxylic acid, preferably a monocarboxylic or a dicarboxylic acid such as citric acid.

3. Means for the treatment according to claim 2, **characterized in that** the acid is contained in the composition in such a quantity that the composition solved in a determined quantity of a aqueous solution
- produces a pH value < 6, preferably < 5 and particutarly preferably < 4.5.

4. Means for the use according to one of the claims 1 to 3, **characterized in that** a hydrogenpersoxosulfate compound is contained as an oxidant.

5. Means for the use according to one of the claims 1 to 4, **characterized in that** the chloric compound is available as an alkaline salt or an alkaline earth metal salt.

6. Means for the use according to one of the claims 1 to 5, **characterized in that** the chloric compound is B-chloramine (natrium-N-chlorbenzolsulfonamide), T-chloramine (p-toluolsulfonchloramine sodium), T-dichloramine (p-toluolsulfondichloramide), halazone (p-dichlorosulfamoyl benzoic acid), dichlorocyanuric acid, trichlorocyanuric acid, TCM (trichloromelamine), 2,3-dichloro-5,5,-dimethyl hydantoin, dichlorogycoluril, succinochlorimide or chloroazodine (N,N'-dichlorodiazodicarbonamidine) or a mixture thereof.

7. Means for the use according to one of the claims 1 to 6, **characterized in that** the composition contains at least potassium hydrogen monopersulfate (KHSO₅) as oxidant and a chloride compound in form of a salt, preferably from the group of the alkaline salts, for example cooking salt (NaCl) as chlorine source.

8. Means for the use according to one of the claims 1 to 7, **characterized in that** the oxidation potential of the oxidant in an aqueous solution is at least in the claimed pH range higher than the oxidation potential of Cl¹⁻/Cl^{o}.

9. Means for the use according to one of the claims 1 to 8, **characterized in that** the pharmaceutical composition is mixed to the adhesive composition in form of a powder solid mixture.

10. Means for the use according to one of the claims 1 to 9, **characterized in that** the composition contains means for the acceleration of dissolution (effervescent salts), for example a compound containing carbonate or bicarbonate such as, for example, sodium carbonate or sodium bicarbonate and an at least stoichiometric quantity of acid.

11. Means for the use according to one of the claims 1 to 10, **characterized in that** the means contains a binding agent and optionally flavourings, colourings and additives such as substances for water softening and fillers and is available as adhesive ointment or gel.

12. Means for the use according to one of the claims 1 to 11, **characterized in that** the adhesive composition contains at least one water soluble polymer, preferably selected from the group of the cellulose derivatives, at least one alkylvinylether/malein anhydride copolymer.

13. Means for the use according to claim 12, **characterized in that** the adhesive composition contains paraffins, greases and/or oils.

14. Means for the use according to one of the claims 1 to 13, **characterized in that** it does not contain any fluoride salts, preferably any fluoride compounds.

## Revendications

1. Agent pour le traitement d'aphtes comprenant :
- une composition adhésive fluide pâteuse qui est apte à adhérer à la muqueuse buccale après application et
- une composition pharmaceutique contenue dans la composition adhésive pour le traitement de l'inflammation,
**caractérisé en ce que** la composition pharmaceutique
- contient au moins un composé chloré organique ou anorganique et
- au moins un agent oxydant qui est sélectionné dans le groupe des composés de persulfate.

2. Agent pour l'utilisation selon la revendication 1, **caractérisé en ce qu'**il contient un acide carboxylique, de préférence un acide monocarboxylique ou un acide dicarboxylique comme l'acide citrique.

3. Agent pour l'utilisation selon la revendication 2, **caractérisé en ce que** l'acide est contenu dans la composition dans une quantité telle que la composition dissoute dans une quantité d'terminée d'une solution aqueuse
- produit une valeur de pH < 6, de préférence < 6 et de manière toute préférée <4,5.

4. Agent pour l'utilisation selon l'une des revendications 1 à 3, **caractérisé en ce qu'**un composé de peroxosulfate d'hydrogène est contenu comme agent oxydant.

5. Agent pour l'utilisation selon l'une des revendications 1 à 4, **caractérisé en ce que** le composé chloré est présent sous forme de sel alcalin ou de sel de métal alcalino-terreux.

6. Agent pour l'utilisation selon l'une des revendications 1 à 5, **caractérisé en ce que** le composé chloré est la chloramine B (N-chlorobenzolsulfonamide de sodium), la chloramine T (p-toluolsulfonchloramine de sodium), la dichloramine T (p-toluolsulfondichloramide), l'halazone (acide benzoïque dichlorosulfamyle), l'acide dichlorocyanurique, l'acide trichlorocyanurique, la TCM (trichloromélamine), 1,3-dichloro-5,5-diméthylhydantoïn, le dichloroglycoluril,le succinochlorimide ou la chloroazodine (N,N'-dichlorodiazodicarbonamidine) ou un mélange de ces substances.

7. Agent pour l'utilisation selon l'une des revendications 1 à 6, **caractérisé en ce que** le composé contient au moins de l'hydrogénomonopersulfate de potassium (KHS05) comme agent oxydant et un composé chloré sous forme de sel, de préférence dans le groupe des sels alcalins, par exemple le sel de cuisine (NaCl) comme source de chlore.

8. Agent pour l'utilisation selon l'une des revendications 1 à 7, **caractérisé en ce que** le potentiel d'oxydation de l'agent oxydant dans une solution aqueuse est au moins dans la plage de pH revendiquée supérieur au potentiel d'oxydation de Cl¹-/Cl^{o}.

9. Agent pour l'utilisation selon l'une des revendications 1 à 8, **caractérisé en ce que** la composition pharmaceutique est mélangée à la composition adhésive sous forme d'un mélange de substance solide poudreuse.

10. Agent pour l'utilisation selon l'une des revendications 1 à 9, **caractérisé en ce que** la composition contient des moyens pour l'accélération de la dissolution (sels effervescents), par exemple un composé qui contient du carbonate ou du bicarbonate comme, par exemple, le carbonate de soude ou le bicarbonate de soude et une quantité au moins stoechiométrique d'acide.

11. Agent pour l'utilisation selon l'une des revendications 1 à 10, **caractérisé en ce que** le moyen contient un liant et en option des substances aromatiques, des colorants et des adjuvants comme des substances pour l'adoucissement de l'eau et des charges et est présent sous forme de pommade adhésive ou de gel.

12. Agent pour l'utilisation selon l'une des revendications 1 à 11, **caractérisé en ce que** la composition adhésive contient au moins un polymère soluble dans l'eau, de préférence sélectionné dans le groupe des dérivés de cellulose, au moins un alkylvinyléther/copolymère d'anhydride maléique.

13. Agent pour l'utilisation selon la revendication 12, **caractérisé en ce que** la composition adhésive contient des paraffines, des graisses et/ou des huiles.

14. Agent pour l'utilisation selon l'une des revendications 1 à 13, **caractérisé en ce qu'**il ne contient pas de sels fluorés, de préférence pas de composés fluorés.
